# EUROPÄISCHE PATENTSCHRIFT

(11) **EP 2 836 456 B1**
(45) Veröffentlichungstag und Bekanntmachung des Hinweises auf die Patenterteilung: **24.08.2016**
(21) Anmeldenummer: 13715953.9
(22) Anmeldetag: 08.04.2013
(51) Int. Cl.: B81C 1/00, A61M 11/02

(54) **VERFAHREN ZUR HERSTELLUNG VON GRABENARTIGEN VERTIEFUNGEN IN DER OBERFLÄCHE EINES WAFERS**
METHOD FOR PRODUCING TRENCH-LIKE DEPRESSIONS IN THE SURFACE OF A WAFER
PROCÉDÉ DE FABRICATION D'ÉVIDEMENTS DU TYPE TRANCHÉE DANS LA SURFACE D'UNE TRANCHE

(30) Priorität: 10.04.2012 EP 12163650
(43) Veröffentlichungstag der Anmeldung: 18.02.2015
(73) Patentinhaber: BOEHRINGER INGELHEIM microParts GmbH, 44227 Dortmund (DE)
(72) Erfinder: KADEL, Klaus, 55216 Ingelheim am Rhein (DE)
(74) Vertreter: Simon, Elke Anna Maria
(86) Internationale Anmeldenummer: PCT/EP2013/057301
(87) Internationale Veröffentlichungsnummer: WO 2013/153028

(56) Entgegenhaltungen:
- WO-A1-99/10922
- WO-A1-2006/097307

## Beschreibung

Die Erfindung bezieht sich auf ein Verfahren zur Herstellung von grabenartigen Vertiefungen in der Oberfläche eines Wafers, insbesondere eines Silizium-Wafers oder eines Glas-Wafers, mittels Plasmaätzen, bei dem die Vertiefungen durch eine wechselweise Passivierung und Ätzung gefertigt werden.

Zur Herstellung von Mikrodüsen zur Abgabe eines Fluids ist es bekannt, diese aus einem Verbund eines Silizium-Wafers und einer darauf aufgebrachten Glasplatte herzustellen. Hierbei werden in die Waferoberfläche mikroporöse Strukturen oder Kanäle, die im Zusammenwirken mit der Glasplatte dann Düsenkanäle ausbilden, geätzt. Alternativ können in eine Oberfläche einer Glasplatte bzw. eines Glas-Wafers mikroporöse Strukturen oder Kanäle geätzt werden, die im Zusammenwirken mit einem Silizium-Wafer oder einer weiteren Glasplatte Düsenkanäle ausbilden. Durch entsprechendes Schneiden des Verbundes aus Siliziumwafer und Glasplatte lassen sich daraus dann durch Sägen Mikrodüsen herausschneiden. Die Mikrodüse kann zwei oder mehr Platten umfassen, von denen mindestens eine erste Platte eine grabenartige Struktur aufweist, wobei die Gräben die Einlassseite und die an der gegenüberliegenden Seite als Auslass vorgesehene(n) Zerstäuberdüse(n) verbinden, während eine andere, im allgemeinen unstrukturierte zweite Platte auf die strukturierte Seite der ersten Platte aufgesetzt und fest mit ihr verbunden ist. Ein Düsenkörper aus drei Schichten kann beispielsweise aus einer strukturierten Siliziumplatte, einer planen Siliziumdeckelplatte und einer dazwischen angeordneten dünnen Glasplatte bestehen.

Gemäß der DE 42 360 37 A1 wird auf der zu strukturierenden Oberfläche eine dünne Schicht aus Silizium thermisch oxidiert. Die Oxidschicht dient später als Maskierung zum Ätzen der Grabenstrukturen. Auf diese Schicht wird im Schleuderverfahren eine lichtsensitive Kunststoffschicht aufgetragen und verfestigt. Die Strukturen werden lichtoptisch mittels Kontaktkopie über eine Maske im Maßstab 1:1 in diese Kunststoffschicht übertragen und entwickelt. Alternativ können die Strukturen mittels Projektionslithographie unter Verwendung von Masken mit vorzugsweise 5-fach vergrößerten Strukturen in die Kunststoffschicht übertragen und entwickelt werden. Die Kunststoffstrukturen dienen im nächsten Prozessschritt als Maskierung für die Strukturierung der Siliziumdioxidschicht. Diese Strukturierung erfolgt durch reaktives Ätzen mit Ionenstrahlen. Bei der Strukturierung der Oxidschicht wird der Kunststoff vollständig abgetragen oder im Anschluss an die Oxidstrukturierung oder Siliziumstrukturierung entfernt. Die so strukturierte Oxidschicht dient nun als Maskierung für das Ätzen der z. B. 5-7 µm tiefen Grabenstrukturen im Silizium. Dabei wird auch die Oxidschicht langsam abgetragen. Am Ende dieses Strukturierungsvorganges befinden sich auf der Siliziumplatte U-förmige bzw. rechtwinklige, kastenförmige Grabenstrukturen, die jedoch in der Draufsicht nahezu beliebige Flächengeometrien aufweisen können. Diese Ätzformen lassen sich sowohl mit isotropen Trockenätzverfahren als auch mit isotropen Naßätzverfahren erzeugen. Mit anisotrop wirkenden Ätzverfahren (sowohl mit reaktivem Ionenplasma als auch mit naßchemischen Mitteln) sind dreieckförmige Düsenquerschnitte aus V-förmigen Grabenstrukturen oder Grabenstrukturen mit senkrechten Kanten von einkristallinen Bodenplatten erzielbar. Die Geometrieform der Gräben kann außerdem über eine Kombination von Ätztechniken und Beschichtungstechniken verändert werden. Hier ergeben sich nahezu beliebige Geometrieformen. Nach der Strukturierung wird die Siliziumplatte gereinigt und das restliche Siliziumdioxid naßchemisch entfernt.

Solche Mikrodüsen finden beispielsweise in medizinischen Inhalatoren Verwendung, deren technische Grundlagen beispielsweise in der WO 91/14468 oder der WO 97/12687 beschrieben sind. Bei diesen Inhalatoren wird die zu vernebelnde Menge der flüssigen Arzneimittelformulierung mittels hohen Drucks von bis zu 500 bar durch eine Mikrodüse mit bevorzugt zwei Düsenausgängen gepresst und dabei in das lungengängiges Aerosol überführt. Auf die genannten Referenzen wird im Rahmen der vorliegenden Erfindungsbeschreibung ausdrücklich in Gänze Bezug genommen.

Es ist Aufgabe der Erfindung ein Verfahren geeignet für eine Mikrodüse sowie einen Zerstäuber mit einer Mikrodüse der eingangs genannten Art zu schaffen, wobei eine selbstreinigende dünne Funktionalisierungsschicht ausgebildet wird.

Erfindungsgemäß wird die Aufgabe bei dem Verfahren dadurch gelöst, dass jede Vertiefung in ihrer Endgeometrie mit einer polytetrafluorethylenartigen Schutzschicht versehen wird.

Unter dem Begriff "polytetrafluorethylenartig" sind CFx- oder CxFy-Polymere zu verstehen die im Rahmen einer Tiefätzung üblicherweise als so genannte Plasmapolymere Verwendung finden. Die Schutzschicht ist chemisch sehr reaktionsträge und weist bei einer niedrigen Oberflächenspannung einen sehr geringen Reibungskoeffizienten auf. Demnach bleiben in den Vertiefungen keine Rückstände von Substanzen, die durchgeleitet werden und die Mikrodüse ist quasi selbst-reinigend.

Die Schutzschicht wird aus dem zur Passivierung verwendeten Plasmapolymer gebildet und durch eine Anpassung des anliegenden Potentials in ihrer Dicke beeinflusst. Bei dem im Zusammenhang mit der Erfindung verwendeten und aus der Praxis sowie der WO 99/10922 bekannten, so genannten Bosch-Prozess erfolgt ein Tiefätzen von Silizium in verschiedenen Teilprozessen, die jeweils durch Ätzen und Passivieren gekennzeichnet sind. Zunächst erfolgt die Abscheidung einer dünnen Plasma-Polymerschicht mit einer Dicke von wenigen nm, die in einem darauf folgenden physikalischen Vorgang an den Oberflächen abgetragen wird. Anschließend erfolgt ein isotropes Ätzen, dem wiederum eine weitere Abscheidung einer Plasma-Polymerschicht zur Passivierung der isotrop geätzten Bereiche für den nächsten Schritt folgt. In den nächsten Schritten wird die Polymerschicht wieder abgetragen und es schließt sich das Ätzen der nächsten Stufe an. Während nach dem Stand der Technik in einem letzten Schritt zum Erreichen der gewünschten Tiefe in der Regel ein Ätzen und eine Abschließende Entfernung des Polymers erfolgt, wird nach der Erfindung in einem letzten Schritt nach dem Erreichen der erforderlichen Tiefe das Plasmapolymer abgeschieden. Um eine relativ große Dicke der Schutzschicht die die Dicke der Schicht zur Passivierung bei weitem übersteigt, zu erzielen, wird das anliegende Potential gegenüber den vorhergehenden Passivierungsschritten verändert, wobei dem Fachmann entsprechende Maßnahmen geläufig sind. Die beschriebene Vorgehensweise ist auch bei einem kombinierten Bosch-Cyro-Prozess, bei dem der Wafer während des Ätzens gekühlt wird, durchführbar.

In Ausgestaltung wird die Schutzschicht vor dem Strippen einer Maskierungsschicht, insbesondere auf der Oberfläche der Vertiefungen, abgeschieden. Durch das Strippen, also Entfernen der Maskierungsschicht, von der Oberfläche wird auch die auf der Maskierungsschicht anhaftende Schutzschicht beseitigt. Die Maskierungsschicht kann insbesondere Fotolack, SiO2, Si3N4 oder Metall umfassen.

Zweckmäßigerweise wird als Gas für die Schutzschicht Trifluormethan (CHF3), Tetrafluoroethylen (C2F4), Octafluorcyclobutan (C4F8) oder eine Mischung dieser Gase mit einem Inertgas verwendet.

Um die Vertiefungen derart zu schließen, dass Kanäle entstehen wird, die die Vertiefungen aufweisende Oberfläche des Wafers nach dem Strippen einer Maskierungsschicht, insbesondere einer Oxidschicht, mit einer Glasplatte durch anodisches Bonden verbunden. Dem Fachmann ist das Bonden beispielsweise aus der US-A-3 397 278 bekannt und ein geeignetes Glas ist beispielsweise ein Alkaliborosilikatglas, das z. B. unter den Handelsnamen Pyrex (#7740 Corning) oder Tempax (Schott) auf dem Markt erhältlich ist. Sind die Vertiefungen in die Oberfläche eines Glas-Wafers eingebracht, können sie selbstverständlich zur Bildung von Kanälen mit einer Siliziumplatte verschlossen werden.

Durch die Schutzschicht ist eine Wechselwirkung von Bestandteilen und Partikeln der durch eine Düsenöffnung der Mikrodüse ausgetragenen Formulierung mit den Grenzflächen der Vertiefungen der Mikrodüse unterbunden. Die freie Energie der Oberfläche der bereichsweise Düse und Düsenkanäle bildenden Vertiefungen und somit die Benetzbarkeit ist in diesem Bereich minimiert, womit eine Verminderung der Immobilisierung von Materialrückständen am Düsenauslass, also unmittelbar im Bereich der Düsenöffnung, einhergeht. Bei der Verwendung der Mikrodüse werden die Materialrückstände ausgetragen und die mit der Schutzschicht versehenen Vertiefungen sind quasi selbstreinigend.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispieles unter Bezugnahme auf die zugehörigen Zeichnungen näher erläutert. Es zeigt:

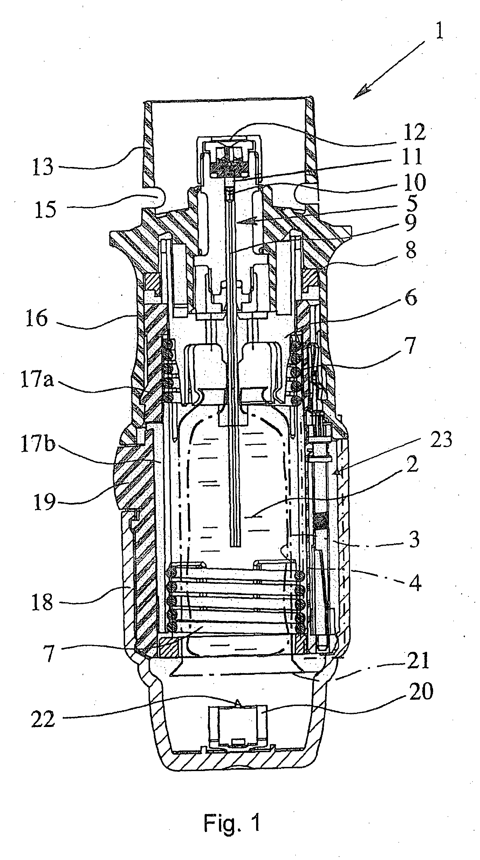

- Fig.1: eine schematische Schnittdarstellung eines erfindungsgemäßen Zerstäubers in einem Auslieferungszustand mit einem eingebauten, verschlossenen Behälter,

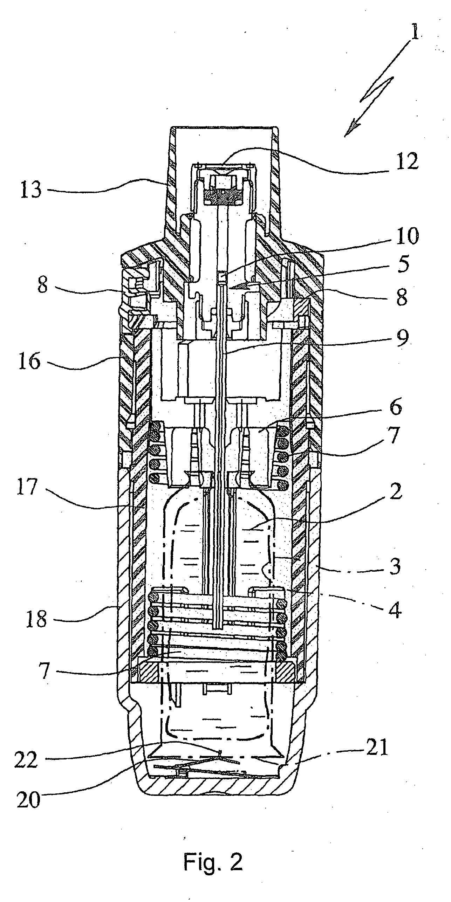

- Fig.2: eine schematische Schnittdarstellung des Zerstäubers nach Fig. 1 mit dem geöffneten Behälter,
- Fig.3 bis Fig.7: Teildarstellungen einer Mikrodüse des Zerstäubers in unterschiedlichen Fertigungsschritten

Der Zerstäuber 1 zur Vernebelung eines Fluids 2, insbesondere eines hochwirksamen Arzneimittels oder dgl., ist vorliegend als tragbarer Inhalator ausgebildet und arbeitet vorzugsweise ohne Treibgas. Bei der Vernebelung des Fluids 2, vorzugsweise einer Flüssigkeit, insbesondere einer ethanolischen bzw. wässrigen Wirkstoffformulierung, wird ein vorzugsweise lungengängiges Aerosol gebildet, das von einem Benutzer bzw. Patienten eingeatmet bzw. inhaliert werden kann. In dem Zerstäuber 1 ist ein im Querschnitt im Wesentlichen zylindrischer Behälter 3 mit dem Fluid 2, insbesondere auswechselbar, eingesetzt, der ein Reservoir für das zu zerstäubende Fluid 2 bildet, das in einem kollabierbaren Beutel 4 im Behälter 3 aufgenommen ist.

Im Weiteren weist der Zerstäuber 1 einen Druckerzeuger 5 zur Förderung und Zerstäubung des Fluids 2 jeweils in einer vorbestimmten, ggf. einstellbaren Dosiermenge auf. Der Druckerzeuger 5 umfasst eine Halterung 6 für den Behälter 3, eine Antriebsfeder 7, ein zur Entsperrung manuell betätigbares Sperrelement 8, ein vorzugsweise als Kapillare ausgebildetes Förderrohr 9 mit einem Rückschlagventil 10 und eine mindestens eine Mikrodüse umfassende Austrittsdüse 12 im Bereich eines Mundstücks 13. Der Behälter 3 ist über die Halterung 6, derart in der Dosiervorrichtung 1 fixiert, dass das Förderrohr 9 in den Behälter 3 eintaucht.

Beim axialen Spannen der Antriebsfeder 7 wird die Halterung 6 mit dem Behälter 3 und dem Förderrohr 9 nach unten bewegt und Fluid 2 aus dem Behälter 3 über das Rückschlagventil 10 in eine Druckkammer 11 des Druckerzeugers 5 gesaugt. Beim anschließenden Entspannen der Antriebsfeder 7 nach der Betätigung des Sperrelements 8 wird das Fluid 2 in der Druckkammer 11 unter Druck gesetzt, indem das Förderrohr 9 bei dem jetzt geschlossenem Rückschlagventil 10 durch Entspannen der Antriebsfeder 7 wieder nach oben bewegt wird und nun als Druckkolben wirkt. Dieser Druck treibt das Fluid 2 durch die Mikrodüsen der Austrittsdüse 12 aus, wobei es in das vorzugsweise lungengängige Aerosol zerstäubt wird. Der Benutzer inhaliert das Aerosol, wobei er über mindestens eine Zuluftöffnung 15 Zuluft in das Mundstück 13 saugt.

Die Dosiervorrichtung 1 weist ein oberes Gehäuseteil 16 und ein demgegenüber drehbares inneres Gehäuseteil 17 mit einem oberen Teil 17a und einem unteren Teil 17b auf, wobei an dem inneren Gehäuseteil 17 ein Gehäuseunterteil 18 mittels eines manuell betätigbaren Halteelementes 19 lösbar befestigt ist. Zum Einsetzen bzw. Auswechseln des Behälters 3 wird das kappenartige Gehäuseunterteil 18 von dem Zerstäuber 1 gelöst.

Das Gehäuseunterteil 18 kann gegenüber dem oberen Gehäuseteil 16 gedreht werden, wobei es den unteren Teil 17b des inneren Gehäuseteils 17 mitnimmt. Dadurch wird die Antriebsfeder 7 über ein nicht dargestelltes, auf die Halterung 6 wirkendes Getriebe in axialer Richtung gespannt. Mit dem Spannen wird der Behälter 3 axial nach unten bewegt, bis der Behälter 3 seine Endlage einnimmt, in der die Antriebsfeder 7 gespannt ist. Bei der Verdrehung des oberen Gehäuseteils 16 gegenüber dem inneren Gehäuseteil 17 wird ein Spindelzählwerk 23 betätigt.

Beim erstmaligen Spannen erfolgt ein bodenseitiges Anstechen bzw. Öffnen des Behälters 3, wobei eine axial wirkende, im Gehäuseunterteil 18 angeordnete Feder 20 am Behälterboden 21 zur Anlage kommt, die mit ihrem Anstechelement 22 den Behälter 3 bzw. eine bodenseitige Versiegelung zur Belüftung ansticht. Während des Zerstäubungsvorgangs wird der Behälter 3 von der Antriebsfeder 7 wieder in seine Ausgangslage axial nach oben bewegt. Der Behälter 3 führt also beim Spannen und Zerstäuben eine Hubbewegung aus.

Die Mikrodüse, umfasst im Wesentlichen eine in einer Oberfläche grabenartige Vertiefungen 24 aufweisende Siliziumplatte 25 und eine die Vertiefungen 24 zur Ausbildung eines Kanalsystems überdeckende Glasplatte 26, die durch anodisches Bonden mit der Siliziumplatte 25 verbunden ist.

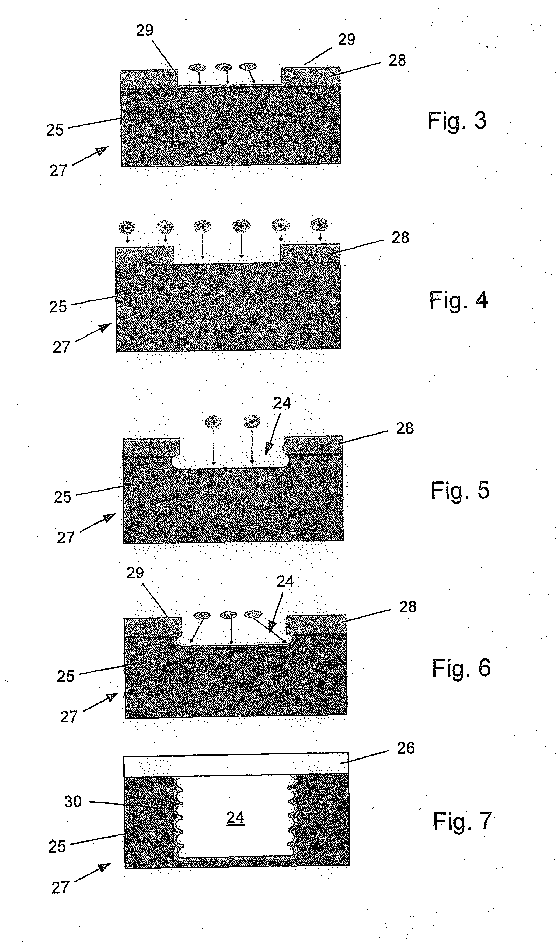

Bei der Fertigung werden die Vertiefungen 24 in die Oberfläche eines Wafers 27 aus Silizium durch ein Tiefätzen in verschiedenen Teilprozessen eingebracht, wobei zunächst eine Maskierungsschicht 28 aufgetragen wird, um den Verlauf der Vertiefungen 26 zu bestimmen. In einem ersten Schritt erfolgt gemäß Fig. 3 die Abscheidung einer dünnen Plasma-Polymerschicht 29 mit einer Dicke von wenigen nm, die nach Fig. 4 in einem zweiten Schritt an den Oberflächen abgetragen wird. In einem dritten Schritt erfolgt entsprechend Fig. 5 ein isotropes Ätzen, dem in einem vierten Schritt nach Fig. 6 eine weitere Abscheidung einer Plasma-Polymerschicht 29 zur Passivierung der isotrop geätzten Bereiche folgt.

Die Schritte wiederholen sich bis die Vertiefung 24 die gewünschte Tiefe aufweist. In einem letzten Schritt, nach dem Erreichen der erforderlichen Tiefe, wird das Plasmapolymer abgeschieden, um eine relativ dicke Schutzschicht 30, die die Dicke der Plasma-Polymerschicht 29 zur Passivierung bei weitem übersteigt, zu erzielen. Als Gas zur Bildung der Schutzschicht 30 wird Trifluormethan (CHF3), Tetrafluoroethylen (C2F4) oder Octafluorcyclobutan (C4F8) verwendet. Nach dem Entfernen der Maskierungsschicht werden die in die Siliziumplatte 25 eingebrachten Vertiefungen 24 mit der Glasplatte 26 verschlossen.

## Patentansprüche

1. Verfahren zur Herstellung von grabenartigen Vertiefungen (24) in der Oberfläche eines Wafers (27), insbesondere eines Silizium-Wafers oder eines Glas-Wafers, mittels Plasmaätzen, bei dem die Vertiefungen (24) durch eine wechselweise Ätzung und Passivierung mit einem polytetrafluorethylenartigen Plasmapolymer gefertigt werden,
**dadurch gekennzeichnet, dass** in einem letzten Schritt, nach dem Erreichen der erforderlichen Tiefe, jede Vertiefung mit einer Schutzschicht (30) aus dem zur Passivierung verwendeten Plasmapolymer versehen wird, wobei die Dicke der Schutzschicht (30) die Dicke die der Plasmapolymerschicht (29) zur Passivierung bei weitem übersteigt und durch eine Veränderung des anliegenden Potentials eingestellt wird.

2. Verfahren nach Anspruch 1, **dadurch gekennzeichnet, dass** die Schutzschicht (30) vor dem Strippen einer Maskierungsschicht (28), insbesondere auf der Oberfläche der Vertiefungen (24), abgeschieden wird.

3. Verfahren nach Anspruch 1, **dadurch gekennzeichnet, dass** als Gas für die Schutzschicht (30) Trifluormethan (CHF3), Tetrafluoroethylen (C2F4) Octafluorcyclobutan (C4F8) oder eine Mischung dieser Gase mit einem Inertgas verwendet wird.

4. Verfahren nach einem der Ansprüche 1 bis 3, **dadurch gekennzeichnet, dass** die die Vertiefungen (24) aufweisende Oberfläche des Wafers (27) nach dem Strippen einer Maskierungsschicht (28), insbesondere einer Oxidschicht, mit einer Glasplatte (26) oder einer Siliziumplatte durch anodisches Bonden verbunden wird.

## Claims

1. Method of producing trench-like depressions (24) in the surface of a wafer (27), particularly a silicon wafer or a glass wafer, by plasma etching, the depressions (24) being produced by alternate etching and passivation with a polytetrafluoroethylene-type plasma polymer, **characterised in that**, in a final step, once the required depth has been reached, each depression is provided with a protective layer (30) made of the plasma polymer used for the passivation, the thickness of the protective layer (30) greatly exceeding the thickness of the plasma polymer layer (29) for the passivation and being adjusted by changing the potential applied.

2. Method according to Claim 1, **characterised in that** the protective layer (30) is deposited before the stripping of a masking layer (28), particularly over the surface of the depressions (24).

3. Method according to Claim 1, **characterised in that** the gas used for the protective layer (30) is trifluoromethane (CHF₃), tetrafluoroethylene (C₂F₄) octafluorocyclobutane (C₄F₈) or a mixture of these gases with an inert gas.

4. Method according to one of claims 1 to 3, **characterised in that** after the stripping of a masking layer (28), particularly an oxide layer, the surface of the wafer (27) comprising the depressions (24) is attached to a glass plate (26) or a silicon plate by anodic bonding.

## Revendications

1. Procédé servant à produire des évidements (24) de type tranchée à la surface d'une tranche (27), en particulier d'une tranche de silicium ou d'une tranche de verre, au moyen d'une gravure par plasma, dans lequel les évidements (24) sont obtenus par une gravure et une passivation réalisées en alternance à l'aide d'un polymère plasma de type polytétrafluoroéthylène,
**caractérisé en ce que** lors d'une dernière étape, après avoir atteint la profondeur requise, chaque évidement est pourvu d'une couche de protection (30) composée du polymère plasma utilisé aux fins de la passivation, l'épaisseur de la couche de protection (30) dépassant largement l'épaisseur de la couche de polymère plasma (29) servant à la passivation et étant ajustée par une modification du potentiel appliqué.

2. Procédé selon la revendication 1, **caractérisé en ce que** la couche de protection (30) est déposée, avant le stripage d'une couche de masquage (28) en particulier sur la surface des évidements (24).

3. Procédé selon la revendication 1, **caractérisé en ce que** du trifluorométhane (CHF3), du tétrafluoroéthylène (C2F4), du octafluorocyclobutane (C4F8) ou un mélange de ces gaz avec un gaz inerte sont utilisés en tant que gaz pour la couche de protection (30).

4. Procédé selon l'une quelconque des revendications 1 à 3, **caractérisé en ce que** la surface, présentant les évidements (24), de la tranche (27) est reliée par un bondage anodique à une plaque de verre (26) à une plaque de silicium après le stripage d'une couche de masquage (28), en particulier d'une couche d'oxyde.
